# EUROPEAN PATENT APPLICATION

(11) **EP 4 241 765 A1**
(43) Date of publication of application: **13.09.2023**
(21) Application number: 22160889.6
(22) Date of filing: 08.03.2022
(51) Int. Cl.: A61K 9/51, A61P 35/00, B82Y 5/00, B82Y 15/00

(54) **GLOBULAR NANOSTRUCTURES HAVING AN ANCHORING LAYER**

(71) Applicant: Spago Nanomedical AB, 223 63 Lund (SE)
(72) Inventor: AXELSSON, Oskar, 243 32 HÖÖR (SE); EKENGARD, Erik, 212 31 MALMÖ (SE); LIU, Yi-Chi, 224 71 LUND (SE)
(74) Representative: Ström & Gulliksson AB

(57) **Abstract**

The present disclosure relates to a plurality of globular nanostructures. Each nanostructure comprises a central part comprising a polymer of monomer residues, wherein the central parts of the nanostructures have a volume average hydrodynamic diameter of 10 nm to 90 nm; and an anchoring layer surrounding the central part, wherein the anchoring layer comprises a polymer of monomer residues according to (RO)₃Si-X-Si(OR)₃, wherein each R is independently selected from the group consisting of a negative charge, H and a covalent bond, wherein at least two R are independently selected from the group consisting of a covalent bond to a monomer residue of the central part and a covalent bond to a monomer residue of the anchoring layer; and X is -(CH₂)ₙ-; and wherein the anchoring layer has a thickness of 1-5 nm. The present disclosure also relates to a method for producing such nanostructures as well as the use of the nanostructures and to pharmaceutical compositions comprising such nanostructures.

## Description

### TECHNICAL FIELD

The present disclosure relates to polymeric nanostructures with applications as precursors to nanostructures with pharmaceutical applications.

### BACKGROUND

The gold standard for cancer treatment is surgery. In cases where surgery alone is not curative, multimodality regimens including chemotherapy and radiation treatment are used. About half of all cancer patients today are treated with radiotherapy, either alone or in combination with other treatments. Radiation delivered as external beams offers a relatively simple and practical approach to causing radiation damage to the tumor. Although the intensity, location and timing for external radiation can be well controlled and modulated, disadvantages associated with this technique include the destruction of normal tissue in the path of the beam as well as damage to tissues surrounding the tumor. The risk of damaging surrounding healthy tissue speaks against external radiotherapy to deeply situated tumors and tumors situated next to vital organs. Furthermore, high radiation doses are frequently required to penetrate the tissue. Moreover, in order to be efficient, external radiotherapy often requires the patients to submit themselves to daily hospital visits over extended periods of time.

Systemic radiotherapy, where a radioactive substance is injected in the blood stream and delivered to the tumor, offers solutions to many of the above-mentioned disadvantages connected with external radiotherapy.

Recent advances in nanotechnology have led to the development of nanocarriers designed for cancer detection and screening, *in vivo* molecular and cellular imaging as well as the delivery of therapeutics. However, despite a large number of publications covering nanosized carriers for cancer therapeutics, relatively few have reached clinical trials, and only a handful are approved by the FDA.

The rationale for nanosized materials being suitable as tumor-targeting radiation carriers is related to the enhanced permeation and retention (EPR) effect. The EPR effect is based on the fact that whereas the capillaries of healthy tissues are virtually impermeable to molecules larger than 3-4 nm, capillaries of fast-growing tumor tissue are much leakier. In addition, solid tumors tend to lack functional lymphatics. Combined, these features limit the removal of extravasated nanomaterials from most solid tumors, leading to an accumulation of nanosized materials in the tumor. Because EPR-mediated drug targeting exclusively relies on the pathological properties of the target tissue, i.e. enhanced leakiness and poor lymphatic drainage, it is generally referred to as passive tumor targeting.

EP2923712A1 discloses nanostructures, having a hydrodynamic diameter of 8-100 nm, which can be used in imaging and/or radiotherapy.

Nanostructures made from silica dissolve rapidly if exposed to aqueous environments at a concentration below the solubility of silicic acid. The dissolution of silica nanostructures occurs through a hydrolytic depolymerization of the silica network, releasing free silicic acid and small oligomers of silicic acid. In general, the silica nanostructures dissolve within a few hours. This is especially pronounced at low concentrations of the nanostructures, such as the concentrations relevant for *in vivo* use of nanostructures.

Dipodal silanes have some of the Si-O-Si-linkages replaced with Si-X-Si-linkages where -X- represents a non-hydrolysable chemical linker. This renders the hydrolytic depolymerization of polymeric networks derived from dipodal silanes slower than the hydrolytic depolymerization of the polymeric network in silica.

US2014275574 discloses the use of dipodal silanes for forming coatings of superior hydrolytic stability on macroscopic objects.

US2012145037 discloses the use dipodal silanes in the fabrication of coatings for glass objects.

Nanoparticles coated with a shell of silica are well known. Often the nanoparticle is a metal or metal oxide nanoparticle. However, the utility of such silica layers as anchoring layers for coatings that render the nanoparticle biocompatible is limited as the anchoring layers tend to dissolve *in vivo.* One object of the present disclosure is to solve this problem.

### SUMMARY

According to a first aspect, the above and other objects are achieved, in full or at least in part by a composition as defined by claim 1. According to this claim, the above object is achieved by a plurality of globular nanostructures, wherein the plurality of globular nanostructures has a dispersity between 1 and 1.8; and wherein each nanostructure comprises a central part comprising a polymer of monomer residues, wherein the central parts of the nanostructures have a volume average hydrodynamic diameter of 10 nm to 90 nm; and an anchoring layer surrounding the central part, wherein the anchoring layer comprises a polymer of monomer residues according to Formula (I)

(RO)₃Si-X-Si(OR)₃ (I)

wherein each R is independently selected from the group consisting of a negative charge, H and a covalent bond, wherein at least two R are independently selected from the group consisting of a covalent bond to a monomer residue of the central part and a covalent bond to a monomer residue of the anchoring layer; and X is -(CH₂)ₙ-, wherein - represents a covalent bond and n is an integer between 1 and 5; and wherein the anchoring layer has a thickness of 1-5 nm.

Preferably, between two and five, of the six groups with potential for crosslinking in the monomer of the anchoring layer form crosslinks, i.e. between 2 and 5 R are independently selected from the group consisting of a covalent bond to a monomer residue of the central part and a covalent bond to a monomer residue of the anchoring layer.

Even more preferred, 3 or 4 R are independently selected from the group consisting of a covalent bond to a monomer residue of the central part and a covalent bond to a monomer residue of the anchoring layer.

A high degree of crosslinking gives an anchoring layer having a high density, which increases the number of attachment points for a coating per surface area of the globular nanoparticle (also referred to as primed nanoparticle as described below). Thus, it is possible to produce a coated nanostructure in which the coating is stably attached to the nanostructure, which in turn increases the biocompatibility and the plasma half-life of the nanoparticle.

The anchoring layer may comprise a homopolymer.

According to one embodiment, the anchoring layer has a thickness of 1.1-2.5 nm, or 1.3-1.6 nm. The anchoring layer may have a thickness of 1.2-2.0, such as 1.3-1.8, such as 1.4-1.6, such as 1.5. Such nanostructures have been demonstrated to be particularly stable under physiological conditions.

According to a further embodiment, 2 to 5 of the R-groups are selected from the group comprising a covalent bond to a monomer residue of the central part and a covalent bond to a monomer residue of the anchoring layer.

According to yet another embodiment, n is 1 or 2. Preferably n=1. Thus, a particularly suitable monomer of the anchoring layer is (RO)₃Si-(CH₂)-Si(OR)₃.

Such a monomer may be accomplished by using the monomer bis-(triethoxysilyl)-methane when producing the anchoring layer.

According to one embodiment, the central part comprises a polymer framework of monomer residues according to Formula (II)

{(OR¹)(OR²)PO}₂-(C){(CH₂)ₘSi(OR³)₃}{(CH₂)ₘSi(OR³)₃} (II)

wherein each R¹ and R² is independently selected from the group consisting of a negative charge and H; each R³ is independently selected from the group consisting of a negative charge, H and a covalent bond to the polymeric framework; wherein at least 3 R³ are bonds to the polymeric framework; and m is an integer between 1 and 5.Such nanostructure can chelate e.g. radioisotopes and can be used in imaging, such as PET or SPECT imaging, and also in the treatment of cancer.

According to a further embodiment, the nanostructures further comprise a coating. The coating may render the nanostructures more biocompatible. Preferably the coating comprises hydrophilic groups. Such coatings are particularly biocompatible.

According to a specific embodiment, the coating comprises polyethylene glycol. Such coatings are particularly biocompatible and stable under physiological conditions. Poly ethylene glycol is also referred to as "PEG".

According to a second aspect of the present disclosure, there is provided a method for producing a plurality of globular nanostructures according to anyone of claims 1 to 7, comprising the steps of:
(1) providing a mixture of precursor nanostructures having a volume average hydrodynamic diameter of 10 nm to 90 nm, each precursor nanostructure comprising a polymer of monomer residues, and monomers according to Formula (III)

   Si{(OR¹)(OR²)(OR³)}-(CH₂)n-Si{(OR⁴)(OR⁵)(OR⁶)} (III)

   wherein R¹, R², R³, R⁴, R⁵, and R⁶ are independently selected from the group consisting of lower alkyls and aryl; and n is an integer between 1 and 5;
   in a mixture of water and a water miscible organic solvent; and wherein the ratio of monomer residues of the precursor nanostructures to monomers according to Formula (III) is 1:0.5 to 1:20; and
(2) heating the mixture of step (1) to a temperature between 20 °C and 150 °C for a time period of 1 to 24 h.

Such a method as proven to yield globular nanostructures having an anchoring layer having a thickness that allows an adequate amount of coating to be applied to nanostructures. A sufficient amount of coating influences the plasma half-life of the nanostructures. If a too small amount of coating is present, the plasma half-life is decreased.

The thickness of the anchoring layer is influenced by the ratio of monomer residues of the precursor nanostructures to monomers according to Formula (III). The ratio of monomer residues of the precursor nanostructures to monomers according to Formula (III) may be 1:1 to 1:10, such as 1:2.5 to 1:7, such as 1:3 to 1:5.

The precursor nanostructures may have a volume average hydrodynamic diameter of 10 nm to 90 nm 12.5 to 50 nm, such as 15 to 30 nm, such as 20 to 25 nm.

According to one embodiment of the method, R¹, R², R³, R⁴, R⁵, and R⁶ are independently selected from the group consisting of methyl and ethyl; and wherein n is 1 or 2. Preferably, n=1. Thus, a suitable monomer for producing the anchoring layer is bis-(triethoxysilyl)-methane.

According to a specific embodiment of the method, the ratio of monomer residues of the precursor nanostructures to monomers according to Formula (III) is 1:0.5 to 1:7; and/or wherein, in step (2), the mixture of step (1) is heated to a temperature between 80 °C and 150 °C for a time period of 2 to 6 h.

Preferably, the ratio of monomer residues of the precursor nanostructures to monomers according to Formula (III) is 1:0.5 to 1:7; and wherein, in step (2), the mixture of step (1) is heated to a temperature between 80 °C and 150 °C for a time period of 2 to 6 h.

According to a third aspect of the present disclosure, there is provided pharmaceutical composition comprising a plurality of globular nanostructures according to the present disclosure, wherein the nanostructures further comprise a coating. Preferably, wherein the coating comprises hydrophilic groups, even more preferably, wherein the coating comprises polyethylene glycol. Such a pharmaceutical composition, can be used to produce a pharmaceutical composition comprising nanostructures comprising radioisotopes. Further, when comprising a radioisotope, may be used for imaging, such as PET and SPECT imaging, and/or for the treatment of cancer.

According to a fourth aspect of the present disclosure, there is provided a pharmaceutical composition for use in the treatment of cancer and/or in imaging, wherein the pharmaceutical composition comprises a plurality of globular nanostructures, wherein the nanostructures further comprise a coating, wherein the globular nanostructures further comprise a radioactive isotope. Preferably, the coating comprises hydrophilic groups, even more preferably, the coating comprises polyethylene glycol.

According to a fifth aspect of the present disclosure, there is provided the use of a plurality of globular nanostructures according to the present disclosure and/or the product of the method according to the present disclosure, as an intermediate in the production of a plurality of globular coated nanostructures.

According to a sixth aspect of the present disclosure, there is provided the use of the plurality of globular nanostructures according to the present disclosure and/or the product of the method according the present disclosure, as an intermediate in the production of a pharmaceutical product.

According to a sixth aspect of the present disclosure, there is provided the use of a pharmaceutical composition according the present disclosure as a carrier of a radioactive isotope.

Other objectives, features and advantages of the present disclosure will appear from the following detailed description, from the experimental data, as well as from the attached claims. It is noted that the disclosure relates to all possible combination of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least one instance of said component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

### DEFINITION OF TERMS

The term "nanostructure" as used herein relates to an entity with a total size in the nanorange, i.e. up to 100 nm. As used herein the term excludes the structures often referred to as "core-shell nanoparticles" or just "nanoparticles" which usually have an inorganic core and an organic coating.

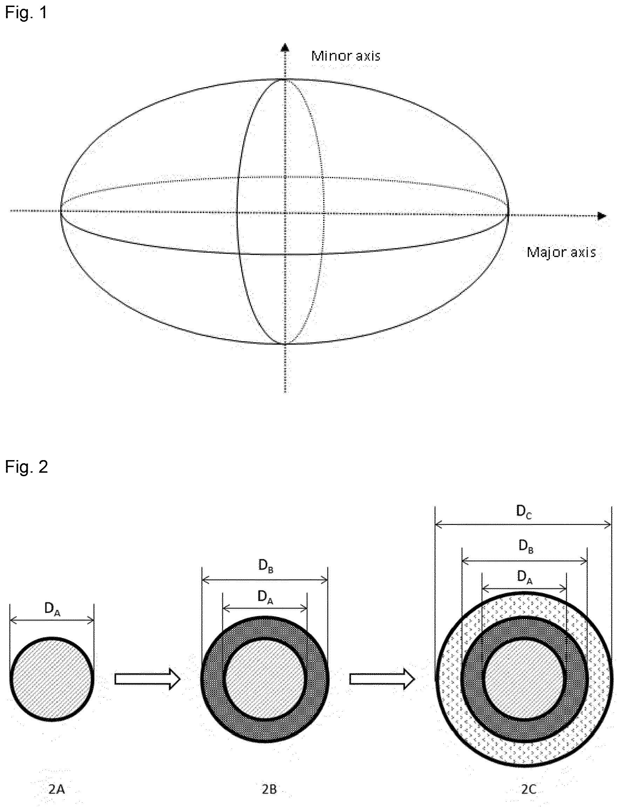

The term "globular" as used herein is meant to describe a shape such that the minor axis is no less than half of the major axis, i.e. the longest axis through the center (point of weight) of the structure is no more than twice the length of the shortest axis through the same point. For an explanatory illustration, not limiting this definition, see Fig. 1.

The term "globular nanostructure" as used herein relates to a nanostructure as discussed above having an essentially globular form or shape. This means that shapes such as flakes, rods, tubes, toroids, chains and ribbons are excluded.

The term "hydrodynamic diameter" as used herein refers to the diameter of the hypothetical hard sphere that diffuses at the same speed as the nanostructure in solution, i. e. the diameter of the equivalent hard sphere as calculated from the diffusion coefficient, according to the Stokes-Einstein equation. The term is also known as "Stokes diameter" or "Stokes-Einstein diameter". Hydration and shape are included in the behavior of the sphere. The diffusion coefficient is in turn calculated from e.g. the time dependent light scattering data obtained by the Dynamic Light Scattering (DLS) technique. Other technical methods to measure the diffusion coefficient of nanostructures are known to one skilled in the art and may be used instead. In those cases, measurements need to be referenced to the DLS-measurement. As a comparison, bovine serum albumin is measured to have a hydrodynamic diameter of 6.9 nm by DLS in aqueous saline at pH 7 and room temperature. Depending on whether the number average, volume average, or scattered intensity average is used, the calculated values may be somewhat different. The volume average is generally the most useful since it shows which nanostructure size the bulk of the material has. The average diameters referred to in this text refer to volume averages as measured at 25 °C in aqueous saline or in 8 % ethylene glycol (v/v) in saline, i.e. volume average hydrodynamic diameter.

The term "DLS" as used herein is an acronym for Dynamic Light Scattering, a particle sizing method, and may also be referred to as Photon Correlation Spectroscopy or Quasi-Elastic Light Scattering. The DLS sizes given as stated in the text and in the claims, if nothing else is specified, refers to the average of the volume weighted peak for a sample measured at 25 °C in aqueous solution with an ionic strength corresponding to 150 mM NaCl, also called saline, or in 8 % ethylene glycol (v/v) in saline.

The term "dispersity" is used where previously the term polydispersity was used. For most applications, it is important to have as narrow a distribution of sizes as possible around the average i.e. a low dispersity. For a sample with completely uniform size the dispersity is 1 and for non-uniform samples it will be larger than 1. The dispersity can be calculated in different ways, either as the ratio of Mw/Mn or similarly Mv/Mn, where Mw is the weight-average molecular weight, Mn is number-average molecular weight, Mv is the volume-average molecular weight. Herein we analogously define dispersity, Dd, for a sample of nanostructures as Dd = dv/dn were dv is the volume-average diameter as measured by DLS and dn is the number average diameter as measured by DLS.

A "monomer" is a molecule that may bind covalently to other molecules of the same kind, (and optionally, other kinds) to form a polymer i.e. a macromolecule composed of several monomer residues. The term "monomer residue" refers to the atoms derived from one monomer unit as incorporated into the larger polymer. Depending on how the monomers link up, all the atoms may be retained or some may be lost as the bonds form.

A "crosslink" refers to a link between two different chains in a polymer. It is usually formed by the reaction of multifunctional monomers (i.e. crosslinkers) added when forming the polymer. Crosslinks may also be introduced e.g. by radiation treatment, chemical means, or heat. For the materials of the present disclosure, which are network polymers of monomers with multiple branching capability, the usual description of degree of crosslinking becomes somewhat unsuitable. For simplicity, we just describe the average number of bonds between monomers. For example: if the monomer has the potential to form six bonds to other molecules and on average three of them are fulfilled, we avoid the term degree of crosslinking and state that we have "on average three bonds to other monomers" or sometimes "on average three bonds out of six possible to other monomers".

The term "crosslinked" refers to a structure formed after the formation of at least one crosslink.

The term "polymeric framework" as used herein relates to a covalently bound group of atoms forming a network structure with multiple crosslinks. Such polymeric frameworks are formed from the linking of suitable monomers and/or oligomers (i.e. a molecular complex consisting of a few monomer residues) via covalent bonds. Some examples of monomers are styrene, propylene, ethylene, tetrafluoroethylene, trifluoroethylene, difluoroethylene, methyl acrylate, ethyl acrylate, hydroxyethyl acrylate, acrylamide, methyl methacrylate, ethyl methacrylate, hydroxyethyl methacrylate, H₂N-(CH₂)_{P}-COOH, where p is 1 -10, 3- aminobenzoic acid, 4-aminobenzoic acid, N-vinyl pyrolidone and silicone precursors like (CH₃COO)₂Si(CH₃)₂. Some examples of polymer frameworks are formed from matching pairs of monomers like terephtalic acid + 1 ,4 diamino benzene, terephtalic acid + ethylene glycol, and HCOO-(CH₂)_{P}COOH + H₂N-(CH₂)_{q}-NH₂, where p and q independently are 1 -10. Oligomers with 2-10 monomer units linked can be used as precursors. Some examples of oligomers different from linked groups of the above monomers are cyclic or poly- cyclic silanes such as hexamethylcyclotrisiloxane, 2,4,6,8- tetramethylcyclotetrasiloxane, and decamethylcyclopentasiloxane. Some examples of crosslinkers are N,N'-methylenebis(acrylamide), 0,0'-methylenebis(acrylic acid), epichlorohydrin, divinylbenzene, 1,3-divinyltetramethyldisiloxane, 1,3-phenylenediisocyanate, 3,3"-biphenyltetracarboxylic acid dianhydride, 1,4-butanedioldivinyl-ether, tetraethoxysilane, oligosilicates such as metasilicate, or silsequioxanes, organosilanes such as bis(triethoxysilyl)methane, bis(triethoxysilyl)ethane, bis(triethoxysilyl)propane, bis(triethoxysilyl)butane, methyl triethoxysilane, ethyl triethoxysilane, and propyl triethoxysilane.

The polymeric framework constitutes the skeleton of the central part of the nanostructure. The skilled person realizes that the random nature of the polymerization process causes the materials to be mixtures of many similar but in most cases not identical branching patterns, crosslink positions and molecular weights.

The term "chelating group" refers to a chemical group with the ability to successfully compete with water in electrostatic binding of a positively charged ion. A single chelating group does not bind very strongly but if several of them surround a positively charged ion, a synergistic strengthening of the binding occurs. This is called chelation.

The term "covalently attached", "covalently linked" and "covalently bound" as used herein are synonymous, and the meaning thereof is well known to the skilled person.

The term "independently selected" as used herein in means that each of the different constituents mentioned before the term is selected from the group following after the term independently or separately from the selection of the other mentioned constituents.

The term "radionuclide" refers to an unstable form of a chemical element that decays radioactively, resulting in the emission of a, β and/or y radiation.

As used herein, the expression "radionuclides for imaging and/or radiotherapy" refers to actinium-225 (²²⁵Ac); copper-62 (⁶²Cu); copper-64 (⁶⁴Cu); copper-67 (⁶⁷Cu); gallium-67 (⁶⁷Ga); gallium-68 (⁶⁸Ga); holmium-166 (¹⁶⁶Ho); indium-111 (¹¹¹In); lead-212 (²¹²Pb); lutetium-177 (¹⁷⁷Lu); radium-223 (²²³ Ra); rhenium-186 (¹⁸⁶Re); rhenium-188 (¹⁸⁸Re); rubidium-82 (⁸²Rb); samarium-153 (¹⁵³Sm); strontium-89 (⁸⁹Sr); technetium-99m (^{99m}Tc³⁺); thallium-201 (²⁰¹TI); thorium-227 (²²⁷Th); yttrium-86 (⁸⁶Y); yttrium-90 (⁹⁰Y); and zirconium-89 (⁸⁹Zr). The expression "a radionuclide for imaging and/or radiotherapy" also encompasses combinations of two or more of the above mentioned radionuclides.

As used herein, the expression "radionuclides for imaging" refers to copper-62 (⁶²Cu); copper-67 (⁶⁷Cu); gallium-67 (⁶⁷Ga); gallium-68 (⁶⁸Ga); indium-111 (11¹In); lutetium-177 (¹⁷⁷Lu); rhenium-186 (¹⁸⁶Re); rubidium-82 (⁸²Rb): technetium-99m (^{99m}Tc³⁺); Thallium-201 (²⁰¹TI)- yttrium-86 (⁸⁶Y) and zirconium-89 (⁸⁹Zr). The expression "a radionuclide for imaging" also encompasses combinations of two or more of the above mentioned radionuclides.

As used herein, the expression "radionuclides for PET imaging" refers to copper-62 (⁶²Cu); gallium-68 (⁶⁸Ga); rubidium-82 (⁸²Rb); yttrium-86 (⁸⁶Y), and zirconium-89 (⁸⁹Zr). The expression "a radionuclide for PET imaging" also encompasses combinations of two or more of the above mentioned radionuclides.

As used herein, the expression "radionuclides for SPECT imaging" refers to gallium-67 (⁶⁷Ga); indium-111 (¹¹¹In); technetium-99m (^{99m}Tc³⁺); lutetium-177 (¹⁷⁷Lu), and thallium-201 (²⁰¹TI). The expression "a radionuclide for SPECT imaging" also encompasses combinations of two or more of the above mentioned radionuclides.

As used herein, the expression "radionuclides for radiotherapy" refers to actinium-225 (²²⁵Ac); copper-64 (⁶⁴Cu); copper-67 (⁶⁷Cu) holmium-166 (¹⁶⁶Ho); lead-212 (²¹²Pb); lutetium-177 (¹⁷⁷Lu); radium-223 (²²³Ra); rhenium-186 (¹⁸⁶Re); rhenium-188 (¹⁸⁸Re); samarium-153 (¹⁵³Sm); strontium-89 (⁸⁹Sr); thorium-227 e²⁷Th) and yttrium-90 (⁹⁰Y). The expression "a radionuclide for radiotherapy" also encompasses combinations of two or more of the above mentioned radionuclides.

As used herein, the expression "radionuclides for PET imaging and radiotherapy" refers to actinium-225 (²²⁵Ac); copper-62 (⁶²Cu); copper-64 (⁶⁴Cu); copper-67 (⁶⁷Cu); gallium-68 (⁶⁸Ga); holmium-166 (¹⁶⁶Ho); lead-212 (²¹²Pb); lutetium-177 (¹⁷⁷Lu); radium-223 (²²³Ra); rhenium-186 (¹⁸⁶Re); rhenium-188 (¹⁸⁸Re)- rubidium-82 (⁸²Rb); samarium-153 (¹⁵³Sm); strontium-89 (⁸⁹Sr); thorium-227 (²²⁷Th); yttrium-90 (⁹⁰Y) and zirconium-89 (⁸⁹Zr). The expression "a radionuclide for PET imaging and radiotherapy" also encompasses combinations of two or more of the above mentioned radionuclides.

As used herein, the expression "radionuclides for SPECT imaging and radiotherapy" refers to actinium-225 (²²⁵Ac); copper-64 (⁶⁴Cu); copper-67 (⁶⁷Cu); gallium-67 (⁶⁷Ga); holmium-166 (¹⁶⁶Ho); indium-111 (¹¹¹In); lead-212 (²¹²Pb); lutetium-177 (¹⁷⁷Lu); radium-223 (²²³Ra); rhenium-186 (¹⁸⁶Re); rhenium-188 (¹⁸⁸Re); samarium-153 (¹⁵³Sm); strontium-89 (⁸⁹Sr); technetium-99m (^{99m}Tc³⁺); thallium-201 (²⁰¹TI); thorium-227 (²²⁷Th) and yttrium-90 (⁹⁰Y). The expression "a radionuclide for SPECT imaging and radiotherapy" also encompasses combinations of two or more of the above-mentioned radionuclides.

The term "oxysilane" as used herein refers to any compounds with one or more oxygen atoms attached to the silicon atom. Non-limiting examples thereof are:

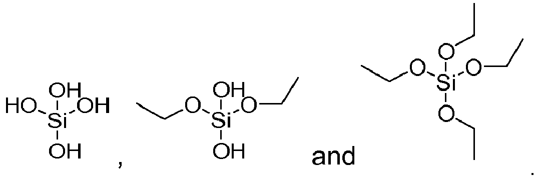

The term "organosilane" as used herein refers to organic compounds containing one or more carbon-silicon bond(s).

The term "alkoxysilane" as used herein refers to any compound with one or more carbon-oxygen-silicon moieties.

The terms "hydrocarbon" and "hydrocarbon chain" are used herein to denote an organic residue consisting of hydrogen and carbon. The hydrocarbon may be fully saturated or it may comprise one or more unsaturations. The hydrocarbon in accordance with the present disclosure may contain any number of carbon atoms between 1 and 50.

The term "alkyl" as used herein refers to a straight or branched hydrocarbon chain fully saturated (no double or triple bonds) hydrocarbon group. The alkyl group may in the present text have 1-15 carbon atoms. Typical alkyl groups include, but are in no way limited to, methyl, ethyl, propyl, isopropyl, butyl, isobutyl, tertiary butyl, pentyl, hexyl and the like.

The term "lower alkyl" as used herein refers to an alkyl having 1-8 carbon atoms.

The term "lower alcohol" as used herein refers to an alcohol having 1-8 carbon atoms.

Numerical ranges: Whenever it is used herein, unless otherwise stated, a numerical range such as "1 to 8" or "1-8" refer to each integer in the given range; e.g., "1 to 8 carbon atoms" and "1-8 carbon atoms" mean that the alkyl group may consist of 1 carbon atom, 2 carbon atoms, 3 carbon atoms, etc., up to and including 8 carbon atoms. There are, however some exceptions which are clear to the skilled persons. In particular, whenever a range is given herein for a molar ratio, such as the Si/P molar ratio in the nanostructures, for a diameter or size, for a pH, for a period of time, for a concentration, for an osmolality or for a temperature, the range includes also all decimal numbers falling within the range, including the upper and lower limits.

As used herein, the term "alkoxy" refers to the formula -OR wherein R is a lower alkyl, e.g. methoxy, ethoxy, n-propoxy, 1 -methyl ethoxy (isopropoxy), n-butoxy, iso-butoxy, sec-butoxy, tert-butoxy, amyloxy, iso-amyloxy and the like. An alkoxy group in accordance with the present disclosure may be optionally substituted.

As used herein the term "aryl" refers to a carbocyclic (i.e. all carbon) ring or two or more fused rings (i.e. rings that share two adjacent carbon atoms) that have a fully delocalized pi-electron system. Examples of aryl groups include, but are not limited to, benzene, naphthalene and azulene. An aryl group in accordance with the present disclosure may be optionally substituted, e.g., phenoxy, naphtha- lenyloxy, azulenyloxy, anthracenyloxy, naphthalenylthio, phenylthio and the like. An aryloxy may be optionally substituted.

As used herein the term "acyl" refers to the functional group RC(=O)-with R being an organic residue.

As used herein he term "coated nanostructure" (or nanostructure having a coating) is used to describe a material that can be produced from the nanostructures of the current disclosure by the addition of one or more layer of additional materials. Often such a coated nanostructure is intended to be used for radioisotope therapy.

As used herein the term "dipodal silane" refers to an organosilane comprising two silicon atoms linked by a non-hydrolysable linker or an essentially non-hydrolysable linker, each silicon atom further substituted such that the silicon atoms independently can react to form bonds to a siloxane network and/or be a part of such a network.

As used herein the term "priming" refers to material applied to another material, object, surface, substrate, interface, or structure with the purpose of aiding, improving, facilitating, enabling, stabilizing, or strengthen the adhesion of, connection to, spreading of, wetting by, reaction with, joining to, surface coverage by, or integration with a third material. Priming may also be used as a verb with the meaning of applying priming (material) (to something) or as a name of the process of applying priming. Which form of the word priming is used is generally clear to one skilled in the art. A material, object, surface, substrate, interface, or structure that has undergone priming can be said to be primed.

BTESM is used as an abbreviation of bis(triethoxysilyl)methane.

As used herein, the term "vol/vol" or "v/v" denotes volume/volume.

### BRIEF DESCRIPTION OF THE FIGURES

By way of example, embodiments of the present teaching will now be described with reference to the accompanying drawings, in which:
Fig. 1 is an explanatory illustration of a globular shape.
Fig. 2 is an explanation of central part, anchoring layer, and coated nanostructure.
Fig. 3 is a scheme of the production process
Fig. 4 Shows a 1H-NMR spectrum of precursor nanostructures (upper trace) and primed globular nanostructures (lower trace).

### DETAILED DESCRIPTION

In short, nanostructures according to the present disclosure have a central part comprising a first type of polymer, and, on the surface of the central part, a reactive layer (the "anchoring layer") of a polymer of a different kind. Such nanostructures comprising both a central part and an anchoring layer are referred to as "primed nanostructures", and nanostructures lacking the anchoring layer, i.e. the central part alone, are referred to as "precursor nanostructures".

The reactive layer, or "anchoring layer", has the purposes of rendering the primed nanostructures reactive or more reactive to derivatization with other materials, henceforth referred to as "coating precursors". The anchoring layer increases the surface density of groups amenable to reaction with the coating precursors on the surface of the nanostructure as compared to the central part without an anchoring layer. After reaction with the reactive groups of the anchoring layer, the coating precursors form a coating on the original nanostructure. The nanostructure comprising a primed nanostructure, i.e. a central part surrounded by an anchoring layer, and a coating are herein referred to as "coated nanostructures". See Fig 2 for a pictorial explanation.

Fig. 2 is a schematic drawing of nanostructures according to the present disclosure. The different components of the nanostructures are described in detail below. In addition, specific features of the different components are discussed.

Fig. 2A depicts the "central part", having an average hydrodynamic diameter D_{A}. Such a nanostructure is also referred to a "precursor nanostructure".

Fig. 2B depicts a nanostructure according to the present disclosure, comprising a "central part" and an "anchoring layer". Such a nanostructure is also referred to a "primed nanostructure". Such a nanostructure has an average hydrodynamic diameter D_{B}. Thus, the thickness of the anchoring layer can be calculated as half the difference between the hydrodynamic diameter of the primed nanostructure and the hydrodynamic diameter of the precursor nanostructure. This corresponds to (D_{B}-D_{A})/2.

Fig. 2C depicts a nanostructure according to the present disclosure, comprising a "central part", an "anchoring layer" and a "coating". Such a nanostructure is also referred to a "coated nanostructure". Such a nanostructure has an average hydrodynamic diameter Dc. Thus, the thickness of the "coating" is (D_{C}-D_{B})/2.

### Primed nanostructures

Compositions comprising primed nanostructures according to the present disclosure will always contain a plurality of said primed nanostructures. The plurality of globular nanostructures can be characterized by statistical measures, such as, but not limited to, average hydrodynamic diameter, average molecular weight, dispersity, density, concentration or size measures such as the percentage passing through certain calibrated filters with nominal cut-off values for the molecular weight.

Specifically, the plurality of globular nanostructures according to the present disclosure comprise primed nanostructures wherein the anchoring layer comprises a crosslinked polymeric network. The polymeric network may be a homopolymer of a single monomer or a copolymer of two or more different monomers.

The primed nanostructures are typically prepared and/or used as a solution, typically as an aqueous solution.

### Central part ("precursor nanostructure")

The average hydrodynamic diameter of the central part corresponds to D_{A} in Fig. 2.

Typically, the central part has an average hydrodynamic diameter between 10 and 90, such as between 12 and 85 nm, such as between 15 and 30, such as 20 and 25.

In one embodiment, the central part has average hydrodynamic diameter between 10 and 25 nm, such as between 15 and 20 nm.

Preferably, the central part comprises chelating groups, such as germinal bisphosphonates.

Preferably, the central part comprises geminal bisphosphonates and organosilanes.

Preferably, the central part comprises a polymer framework of monomer residues according to Formula (II)

{(OR¹)(OR²)PO}₂-(C){(CH₂)ₘSi(OR³)₃}{(CH₂)ₘSi(OR³)₃} (II)

wherein each R¹ and R² is independently selected from the group consisting of a negative charge and H; each R³ is independently selected from the group consisting of a negative charge, H and a covalent bond to the polymeric framework; wherein at least 3 R³ are bonds to the polymeric framework; and m is an integer between 1 and 5.

### Anchoring layer

The thickness of the anchoring layer affects the hydrolytic stability and the plasma half-life of the nanostructure *in vivo.* Nanostructures with a too thin anchoring layer, typically below 1 nm have a shorter plasma half-life than nanostructures having an anchoring layer of more than 1 nm (see Example 9). Thus, nanostructures with a too thin anchoring layer are inferior when used as precursors for coated nanostructures which are to be used in-vivo as described below.

The thickness of the anchoring layer is between 1 and 5 nm.

In one embodiment, the thickness of the anchoring layer of the current disclosure is between 1.1 and 2.5 nm, such as between 1.2 and 2 nm, such as between 1.3 and 1.6 nm, such as between 1.4 and 1.5 nm.

The degree of crosslinking of the polymer of the anchoring layer is unusually high for random polymers such as on average more than two bonds per monomer; or more than three bonds per monomer. Even such high degrees as 4 to 5 may be contemplated for some polymeric frameworks according to the present disclosure.

It is obvious to the person skilled in the art that even if monomers with potential for crosslinking or branching are used as monomers to produce the central part, not all of the potential would be utilized but instead leave residual groups with potential for crosslinking for reaction with a precursor of the anchoring layer. Thus, in nanostructures according to the present disclosure, the anchoring layer is covalently bound to the central part.

In one embodiment, the degree of crosslinking achieved for the anchoring layer is on average of between three and five bonds per monomer.

In another embodiment, the anchoring layer comprises a homopolymer.

Preferably, between two and five of the six groups with potential for crosslinking in the monomer actually form crosslinks.

A high degree of crosslinking gives an anchoring layer having a high density.A high degree of crosslinking may be achieved by the method disclosed below. In Example 12, it was observed that the density of the anchoring layer (1.9 g/ cm³) is, although higher than most organic solids, not as high as the bulk density of amorphous silica (2.65 g/cm³, htt^{p}s://en.wiki^{p}edia.or^{g}/wiki/Silicon dioxide). The atom density of silica, assuming that it is pure Si0₂, is as high as 80 atoms/nm³. For the anchoring layer, where there is a CH₂ instead of an oxygen linking a pair of silicon atoms it is estimated that the atom density for the anchoring layer is close to that (77 non-hydrogen atoms/nm³) if there are in average four bonds to other monomer residues. The evaluation of the density is described in Example 12. According to the present disclosure, bis-(trialkoxysilyl)-alkanes are used for the formation of the anchoring layer of the nanostructures. Thus, the anchoring layer comprises a polymer of monomer residues according to Formula (I)

(RO)₃Si-X-Si(OR)₃ (I)

wherein each R is independently selected from the group consisting of a negative charge, H and a covalent bond, wherein at least two R are independently selected from the group consisting of a covalent bond to a monomer residue of the central part and a covalent bond to a monomer residue of the anchoring layer; and X is -(CH₂)ₙ-, wherein - represents a covalent bond and n is an integer between 1 and 5.

Preferably, 2 to 5 of the R-groups are selected from the group comprising a covalent bond to a monomer residue of the central part and a covalent bond to a monomer residue of the anchoring layer. Thus, as stated above, the anchoring layer may comprise a homopolymer of monomer residues according to Formula (I), where between 2 and 5 of the R-groups, preferably 3 or 4, have formed covalent bonds to either other monomers of the anchoring layer or to the central part.

Preferably, the alkoxysilanes are separated by one or two carbon atoms, i.e. n is 1 or 2. Preferably, n is 1.

A particularly suitable monomer for the anchoring layer is bis-(triethoxysilyl)-methane (see Example 1-b). A nanoparticle having such an anchoring layer has been shown to have a plasma half-life of (see Examples 4 and 9).

Optionally, other functional groups may be present on the carbon atom(s) between the silanes.

Typically, the anchoring layer serves as the attachment layer for a further polymer layer referred to as a "coating" or "coating layer". The coating (described in more detail below) i.a. increases the biocompatibility of the nanostructures.

The anchoring layer of the primed nanostructures allows a higher coating density than coating directly on the precursor nanostructures. An insufficient coating density can result in nanostructures with poor suspension stability, leading to aggregation and/ or precipitation of the nanostructures under conditions where nanostructures with a higher coating density remain in solution without aggregating (see Example 7).

Another advantage of the primed nanostructures is their stability towards degradation. In a hydrolytic environment, such as in a pharmaceutical formulation or inside a living organism they are significantly more stable than nanostructures having a coating formed directly on the precursor nanostructures. In Examples 8 and 9 this is shown for an aqueous solution and in an in-vivo experiment, respectively.

To illustrate that the anchoring layer according to the present disclosure is superior to well-established methods, a material where the coating layer is applied to a nanostructure where the anchoring layer is amorphous silica, derived from tetraethyl orthosilicate, was prepared (see Example 6), and in Examples 8 and 9 is shown that the material has poor stability in aqueous solution and in-vivo compared to nanostructures having an anchoring layer according to the present disclosure.

### Specific embodiments of primed nanostructures

In one embodiment, the central part has a diameter between 10 and 85 nm and the anchoring layer is between 1 and 5 nm.

In another embodiment, the central part has a diameter between 10 and 25 nm and the anchoring layer is between 1 and 2.5 nm.

In yet another embodiment, the central part has a diameter between 15 and 20 nm and the anchoring layer is between 1 and 2 nm.

In a further embodiment, the central part has a diameter between 15 and 20 nm and the anchoring layer is between 1.3 and 1.6 nm.

In yet another embodiment, the dispersity of the nanostructures is below 1.35.

In one embodiment, the dispersity of the nanostructures is below 1.25.

In one specific embodiment, the precursor nanostructure has been synthesized by polycondensation of monomers comprising geminal bisphosphonates and organosilanes.

In a particular embodiment, the central part, i.e. the precursor nanoparticle, comprises a polymer framework of monomer residues according to Formula (II)

{(OR¹)(OR²)PO}2-(C){(CH2)mSi(OR³)3}{(CH2)mSi(OR³)3} (II)

wherein each R¹ and R² is independently selected from the group consisting of a negative charge and H; each R³ is independently selected from the group consisting of a negative charge, H and a covalent bond to the polymeric framework; wherein at least 3 R³ are bonds to the polymeric framework; and m is an integer between 1 and 5. Preferably, m is 3.

In this embodiment, the anchoring layer comprises a polymer of monomer residues according to Formula (I)

(RO)3Si-(CH2)n-Si(OR)3 (I)

wherein each R is independently selected from the group consisting of a negative charge, H and a covalent bond, wherein at least two R, preferably 3-4, are independently selected from the group consisting of a covalent bond to a monomer residue of the central part and a covalent bond to a monomer residue of the anchoring layer, and n is an integer between 1 and 5, preferably n=1.

The anchoring layer has a thickness of 1-5 nm, preferably between 1.2-2 nm.

The dispersity of the globular nanostructures is lower than 1.35, preferably lower than 1.25

### Method for producing primed nanostructures

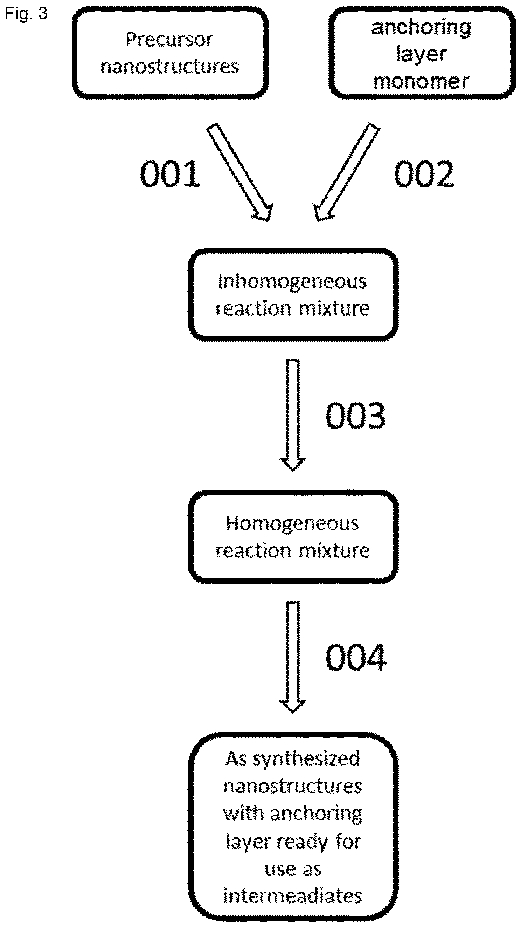

The process for producing primed nanostructures from precursor nanostructures is outlined in Fig. 3. In order to produce primed nanostructures of a quality suitable for large scale production and of a specific size and size distribution, specific process conditions have to be met, as further elaborated below. It is conceivable to mix two, three or several different monomers in any chemically compatible monomer combination, either by mixing the monomers prior to polymerization, or by grafting one polymer to another.

The process to obtain the nanostructures of the current disclosure involves mixing the precursor nanostructures in solution together with the monomer precursor of the anchoring layer for an extended period of time, such as during 1 to 24 h or during 2 to 6 h, at a temperature between 20 °C and 150 °C (steps 003 and 004 in Fig.3).

The solvent mixture is water and a water miscible organic solvent. Preferably, the solvent is a lower alcohol, such as diols, such as ethylene glycol, propylene glycol, or 1,3-propane diol. Especially, water/ethylene glycol in a ratio of 2:8 to 1:9 is particularly preferred. Mixtures of water with two or more water miscible solvents can also be used.

The heating can occur either in a sealed, pressure resistant vessel or at atmospheric pressure. If the heating is conducted at atmospheric pressure, adequate measures to minimize the evaporation of volatile components of the reaction mixture, such as the use of an efficient condenser, are advantageous.

To achieve the desired thickness of the anchoring layer, a specific starting concentration of the silane monomer according to Formula (III) has to be combined with a specific concentration of the precursor nanostructure. In Table 1 of Example 11 is shown data that are sufficient for one skilled in the art to produce high-quality primed nanostructures of a given thickness between 0.6 and 2.5 nm for precursor nanostructures between 12.5 and 23 nm. According to the present disclosure, the ratio of monomer residues of the precursor nanostructures to monomers according to Formula (III) is 1:0.5 to 1:20. Preferably, the ratio is 1:1 to 1:10, such as 1:2.5 to 1:7, such as 1:3 to 3.

The thickness of the anchoring layer is dependent on both the size of the precursor nanostructures and the amount of anchoring layer precursor monomers. The size of the precursor nanostructure is dependent on the number of monomer residues constituting the precursor nanostructure. For a given ratio of the number of monomer residues in the precursor nanostructure solution to the amount of anchoring layer precursor monomers according to Formula (III), the resulting anchoring layer is thicker for larger precursor nanostructures.

In one embodiment of the method, a mixture of 5-25 % of water in a lower alcohol is used to dissolve the precursor nanostructures. Preferably, a mixture of 5-25 % of water in ethanol, 1- or 2-propanol or 1,2- or 1,3-propanediol or ethylene glycol is used to dissolve the precursor nanostructures. Even more preferably, a mixture of 5-25 % of water ethylene glycol is used to dissolve the precursor nanostructures.

The mixture is heated to a temperature between 20 °C and 150 °C for a time period of 1 to 24 h. It has been found to be advantageous to use temperatures higher than room temperature for this process such as temperatures between 80 and 150 °C, for the reaction to proceed at a practical rate.

In one embodiment, the mixture is heated to a temperature of 90-120 °C for a time period of 2 to 6 hours.

In another embodiment, the conditions of this process are a temperature of 95-105 °C and a duration from 3 to 5 hours.

In a further embodiment, the mixture is heated to a temperature of 100 °C for a time period of 4 hours.

The anchoring layer precursor monomers may be immiscible with the precursor nanostructure solution. In such cases, the mixture is heated with stirring until a homogenous solution is formed.

The anchoring layer precursor monomers may be finely dispersed in the precursor nanostructure solution by agitation. In such cases, when the anchoring layer precursor monomer is finely dispersed in the precursor nanostructure solution, the mixture becomes homogeneous before the process is complete. In cases when the anchoring layer precursor is finely dispersed in the precursor nanostructure solution and the temperature of the precursor nanostructure solution is higher than room temperature, the mixture often becomes homogeneous quickly, such as within 60 minutes, or within 30 minutes, or within 15 minutes, or within 10 minutes.

In one embodiment, the solution resulting from this process is further processed by filtration.

### Monomers for the anchoring layer

The silanes used to form the anchoring layer have the generic structure according to Formula (III):

Si{(OR¹)(OR²)(OR³)} (CH₂)ₙSi{(OR⁴)(OR⁵)(OR⁶)} (III)

wherein R¹, R², R³, R⁴, R⁵, and R⁶ are independently selected from the group consisting of lower alkyls and aryl; and n =1-5.

The reactivity of the trialkoxy silanes in the above monomers towards polymerization varies with the identity of the R¹⁻⁶ groups. Methyl and ethyl, in particular the latter, have been found to be particularly suitable for yielding the structures of the present disclosure. However, it is conceivable to use any other lower alkyl group, aryl, or the alkoxy groups may be changed to silyl amide, acyl, silylfluoride, or silylchloride.

In one embodiment, R¹, R², R³, R⁴, R⁵, and R⁶ in Formula (III) are independently methyl or ethyl groups and n = 1.

In a particular embodiment, R¹, R², R³, R⁴, R⁵, and R⁶ in Formula (III) are ethyl groups and n = 1.

In another particular embodiment, R¹, R², R³, R⁴, R⁵, and R⁶ in Formula (III) are methyl groups and n = 1.

In one embodiment, R¹, R², R³, R⁴, R⁵, and R⁶ in Formula (III) are isopropyl groups and n = 1.

In another embodiment, R¹, R², R³, R⁴, R⁵, and R⁶ in Formula (III) are ethyl groups and n = 2.

In yet another embodiment, R¹, R², R³, R⁴, R⁵, and R⁶ in Formula (III) are methyl groups and n = 2.

In a specific embodiment, R¹, R², R³, R⁴, R⁵, and R⁶ in Formula (III) are ethyl groups and n = 1 and the precursor nanostructure comprises chelating groups.

In another specific embodiment, R¹, R², R³, R⁴, R⁵, and R⁶ in Formula (III) are ethyl groups and n = 1 and the precursor nanostructure comprises geminal bisphosphonates. Preferably, the precursor nanostructure, the central part, i.e. the precursor nanoparticle, comprises a polymer framework of monomer residues according to Formula (II) as described above.

### Method for producing precursor nanostructures followed by priming

The globular primed nanostructure may be produced by a process comprising the steps of forming a precursor nanostructure followed by contacting the resulting precursor nanostructures with a precursor of the anchoring layer as described above. The process is outlined in Fig. 3.

Specifically, in one embodiment, the precursor nanostructure is formed by a hydrolytic polymerization of a disilane according to Formula (IV)

{(OR⁷)2PO}2-(C){(CH2)mSi(OR⁸)3}2 (IV)

wherein R⁷ are independently selected from the group consisting of negative charge, H, lower alkyl, and aryl; and R⁸ are independently selected from the group consisting of lower alkyl or aryl, and m is an integer between 1 and 5. The resulting precursor nanostructures are contacted with precursor monomers of the anchoring layer under the conditions described above, whereby the anchoring layer is formed and also covalently linked to the central part (the precursor nanostructure).

In one embodiment, the hydrodynamic diameter of said precursor nanostructure is between 10 and 90 nm, such as between 10 and 30 nm, such as between 15 and 20 nm.

The globular primed nanostructure may be formed by contacting the solution of the precursor nanostructure with the priming precursor in the same vessel as the precursor nanostructure has been synthesized in, without any intermediate processing steps.

The solution of the precursor nanostructure may be further processed prior to contacting with the precursor of the anchoring layer. The solution of the precursor nanostructure may for example be further processed by filtration prior to contacting with the precursor of the anchoring layer. Alternatively, the solution of the precursor nanostructure may be further processed by filtration and solvent exchange prior to contacting with the precursor of the anchoring layer. The solution of the precursor nanostructure may be further processed by chromatography prior to contacting with the precursor of the anchoring layer.

In one embodiment, R¹, R², R³, R⁴, R⁵, and R⁶ in Formula (III) are ethyl groups and n = 1 and the precursor nanostructure was synthesized by polycondensation of monomers comprising geminal bisphosphonates and organosilanes.

In one embodiment, R¹, R², R³, R⁴, R⁵, and R⁶ in Formula (III) are ethyl groups and n = 1 and the precursor nanostructure was synthesized from a precursor according to Formula (IV)

{(OR⁷)₂PO}₂-(C){(CH₂)ₘSi(OR⁸)₃}₂ (IV)

wherein R⁷ and R⁸ are independently selected from the group consisting of lower alkyls, substituted and unsubstituted aryls; and m is an integer between 1 and 5.

In one embodiment, R¹, R², R³, R⁴, R⁵, and R⁶ in Formula (III) are ethyl groups and n = 1 and the precursor nanostructure was synthesized from a precursor comprising 1,7-bis(triethoxysilyl)-4,4-bis(dimethoxyphosphonato)-heptane.

In one embodiment, R¹, R², R³, R⁴, R⁵, and R⁶ in Formula (III) are ethyl groups and n = 1 and the precursor nanostructure comprises monomer residues according to Formula (IV)

{(OR⁷)2PO}2-(C){(CH2)mSi(OR⁸)3}2 (IV)

wherein each R⁷ is independently selected from the group consisting of a negative charge and H; at least 3 of the R⁸-groups represent a bond to the polymeric framework; and the remaining R⁸-groups are independently selected from the group consisting of a negative charge and H; and m is an integer between 1 and 5.

In one embodiment, R¹, R², R³, R⁴, R⁵, and R⁶ in Formula (III) are ethyl groups and n = 1 and the precursor nanostructure comprises monomer residues according to Formula (IV)

{(OR⁷)2PO}2-(C){(CH2)mSi(OR⁸)3}2 (IV)

wherein each R⁷ is independently selected from the group consisting of a negative charge and H; at least 3 of the R⁸-groups represent a bond to the polymeric framework; and the remaining R⁸-groups are independently selected from the group consisting of a negative charge and H; and m = 3.

In one embodiment, the precursor nanostructure is synthesized from a precursor comprising 1,7-bis(triethoxysilyl)-4,4-bis(dimethoxyphosphonato)-heptane by heating the precursor to the precursor nanostructure in a mixture of a lower alcohol and water and the globular primed nanostructure is formed by contacting the resulting solution with bis(triethoxysilyl)methane at between 90 °C and 120 °C for between 2 hours and 6 hours.

In one embodiment, the precursor nanostructure is synthesized from a precursor comprising 1,7-bis(triethoxysilyl)-4,4-bis(dimethoxyphosphonato)-heptane by refluxing the precursor to the precursor nanostructure in aqueous ethylene glycol and the globular primed nanostructure is formed by contacting the resulting solution with 2.5 equivalents (relative to 1,7-bis(triethoxysilyl)-4,4-bis(dimethoxyphosphonato)heptane) of bis(triethoxysilyl)methane at between 95 °C and 105 °C for between 3 hours and 5 hours.

In one embodiment, the precursor nanostructure has a hydrodynamic diameter between 15 nm and 20 nm and is synthesized from a precursor comprising 1,7-bis(triethoxysilyl)-4,4-bis(dimethoxyphosphonato)-heptane by refluxing the precursor to the precursor nanostructure in 90% aqueous ethylene glycol for 48 hours and the globular primed nanostructure is formed by contacting the resulting solution with 2.5 equivalents (relative to 1,7-bis(triethoxysilyl)-4,4-bis(dimethoxyphosphonato)heptane) of bis(triethoxysilyl)-methane at 100 °C for 4 hours resulting in an anchoring layer of thickness between 1 nm and 2 nm.

In one embodiment, the precursor nanostructure is synthesized from a precursor comprising 1,7-bis(triethoxysilyl)-4,4-bis(dimethoxyphosphonato)-heptane by heating the precursor to the precursor nanostructure in a mixture of a lower alcohol and water and the globular primed nanostructure is formed by contacting the resulting solution with 1,2-bis(triethoxysilyl)ethane at between 90 °C and 120 °C for between 2 hours and 6 hours.

### Use of the primed nanostructures as precursors for coated nanostructures

The primed nanostructures according to the present disclosure may be used as precursors for other materials, which are suitable for various applications.

One such application is as precursors to coated nanostructures. Such coated nanostructures may be for in-vivo use.

Thus, the nanostructures of the current disclosure may be further derivatized to render them suitable for in-vivo use. Typically, the primed nanostructures are modified to comprise a coating. Preferably, the coating comprises hydrophilic groups, which makes the nanostructures more biocompatible. In a specific embodiment, the coating comprises polyethylene glycol.

The coated nanostructures are suitable as nanocarriers for radionuclide imaging and/or therapy.

The coated nanostructures may be used as an intravenous imaging agent and/or radiotherapeutic agent. In such applications, the average hydrodynamic diameter of the final coated nanostructures is between 15 and 100 nm, preferably between 20 and 50 nm, and most preferably between 25 and 35 nm. The hydrodynamic diameter of the final coated nanostructures corresponds to Dc in Figure 2c.

### Pharmaceutical compositions

The present disclosure also relates to a pharmaceutical composition comprising a plurality of globular nanostructures according to the present disclosure, preferably wherein the globular nanostructures further comprise a coating. Such a pharmaceutical composition is preferably prepared as a solution, preferably an aqueous solution.

In one embodiment, the pharmaceutical composition further comprises one or more excipients, such as anti-oxidants and/or a buffer.

The pharmaceutical composition may be used as such. Alternatively, the nanostructures in the pharmaceutical composition chelate a metal. In certain cases, the nanostructures in the pharmaceutical composition chelate a radioactive isotope.

Typically, when used for treatment of cancer and/or imaging, the globular nanostructures further comprise a radioactive isotope. Depending on the application, different radioactive isotopes are used.

The composition may comprise nanostructures comprising one single radioactive isotope or two or more different types of radioactive isotopes. Radioactive isotopes, also referred to as radionuclides, suitable for different applications have been listed above.

In one embodiment, the pharmaceutical composition is used for imaging.

A pharmaceutical composition according to the present disclosure used for SPECT imaging typically comprises gallium-67 (⁶⁷Ga); indium-111 (¹¹¹In); technetium-99m (^{99m}Tc³⁺); lutetium-177 (¹⁷⁷Lu), and/or thallium-201 (²⁰¹TI).

A pharmaceutical composition according to the present disclosure used for PET imaging typically comprises copper-62 (⁶²Cu); gallium-68 (⁶⁸Ga); rubidium-82 (⁸²Rb); yttrium-86 (⁸⁶Y), and/or zirconium-89 (⁸⁹Zr).

A pharmaceutical composition according to the present disclosure is used for radionuclide therapy typically comprise actinium-225 (²²⁵Ac); copper-64 (⁶⁴Cu); copper-67 (⁶⁷Cu); holmium-166 (¹⁶⁶Ho); lead-212 (²¹²Pb); lutetium-177 (¹⁷⁷Lu); radium-223 (²²³Ra); rhenium-186 (¹⁸⁶Re); rhenium-188 (¹⁸⁸Re); samarium-153 (¹⁵³Sm); strontium-89 (⁸⁹Sr); thorium-227 (²²⁷Th) and/or yttrium-90 (⁹⁰Y).

In one embodiment, the pharmaceutical composition is used for radionuclide therapy of metastatic cancer.

The pharmaceutical composition may be used to prepare a pharmaceutical composition comprising nanostructures comprising radioisotopes. Such a pharmaceutical composition is prepared by contacting a solution of nanostructures with a solution comprising a radionuclide. Preferably, the nanostructures comprise a coating, preferably a coating comprising polyethylene glycol-moieties.

The pharmaceutical composition comprising chelated radioisotope(s) may be prepared by contacting a solution of nanostructures with a solution of comprising a radionuclide at or close to the point of use.

Alternatively, the pharmaceutical composition comprising chelated radioisotope(s) is prepared by contacting a solution of nanostructures with a solution of comprising a radionuclide at a location separated from the point of use by some distance and the pharmaceutical composition comprising chelated radioisotope(s) is transported to the point of use quite quickly, such as within a month, such as within two weeks, such as within a week, such as within 3 days, such as within a day.

### EXAMPLES

### General experimental conditions

Materials, reagents and solvents were obtained from commercial sources and were used without further purification unless otherwise noted. Solvents were of reagent grade or similar if not otherwise noted.

1,7-bis-(triethoxysilyl)-4,4-bis-(dimethylphosphonato)-heptane was prepared according to published procedures (WO2013041623A1, Example 3).

DLS was measured using a Malvern Instruments Zetasizer Nano ZEN3600 and processed using the general process setting in the Zetasizer software.

### Example 1. Adding an anchoring layer to precursor nanostructures to form primed nanostructures

### Example 1-a. Large scale addition of an anchoring layer to precursor nanostructures to form primed nanostructures, using bis(triethoxysilyl)methane (BTESM) as the anchoring layer precursor

A 5-liter jacketed reactor equipped with a mechanical stirrer, a temperature probe, and a connection to a N₂/vacuum manifold was charged with 4338 g of a solution of precursor nanostructures with an average diameter of 17.6 nm and a phosphorus concentration of 70 mM, formed by refluxing 93.60 g of 1,7-bis-(triethoxysilyl)-4,4-bis-(dimethylphosphonato)-heptane in 4501 g of 90.0 % (v/v) aqueous ethylene glycol for 48 hours. The mantle temperature was set to 100 °C and when the inner temperature was above 99 °C, 116.48 g of bis(triethoxysilyl)methane was added. The mixture was stirred at 300 RPM for 5 minutes, after which the mixture was homogenous and stirring speed lowered to 150 RPM. Heating was continued for 4 h before the solution was cooled to ambient temperature and filtered through double glass fiber filters. Average diameter (DLS) = 19.7 nm, [P](ICP-OES) = 64 mM, [Si](ICP-OES) = 225 mM. Thus, the thickness of the anchoring layer was 1.05 nm.

### Example 1-b. Small scale addition of an anchoring layer to precursor nanostructures to form primed nanostructures, using bis(triethoxysilyl)methane as the anchoring layer precursor

A 20 ml reaction vial equipped with a magnetic stir bar was charged with 10 ml of a solution of precursor nanostructures with an average diameter of 17.5 nm and a phosphorus concentration of 136 mM, formed by heating 7.91 g of 1,7-bis-(triethoxysilyl)-4,4-bis-(dimethylphosphonato)-heptane in 146 ml ethylene glycol and 36.5 ml water to 125 °C for 264 hours. The vial was heated in a preheated 100 °C oil bath for 15 minutes before 553 mg of bis(triethoxysilyl)methane (1.63 mmol, 2.5 equivalent relative to the nominal amount of 1,7-bis-(triethoxysilyl)-4,4-bis-(dimethylphosphonato)-heptane in the solution) was added. The mixture was stirred vigorously for 10 minutes after which the solution was homogenous and the stirring speed lowered. After 4 hours of heating the vial was removed from the oil bath. Average diameter (DLS) = 20.5 nm, [P](ICP-OES) = 125 mM, [Si](ICP-OES) = 488 mM. Thus, the thickness of the anchoring layer was 1.5 nm.

### Example 1-c. Small scale addition of an anchoring layer to precursor nanostructures to form primed nanostructures, using 1,2-bis(triethoxysilyl)-ethane as the anchoring layer precursor

A 25 ml round bottom flask equipped with a magnetic stir bar was charged with 10 ml of a solution of precursor nanostructures with an average diameter of 16.2 nm and a phosphorus concentration of 71 mM, formed by heating 5.82 g of 1,7-bis-(triethoxysilyl)-4,4-bis-(dimethylphosphonato)-heptane in 250 ml 90.0 % (v/v) aqueous ethylene glycol reflux (140 °C) for 90 hours. The flask was heated in a preheated 100 °C oil bath for 10 minutes before 315 mg of 1,2-bis(triethoxysilyl)ethane (0.88 mmol, 2.5 equivalents relative to 1,7-bis-(triethoxysilyl)-4,4-bis-(dimethylphosphonato)-heptane) was added. The mixture was stirred vigorously for 15 minutes after which the solution was homogenous and the stirring speed lowered. After 4 hours of heating the flask was removed from the oil bath. Average diameter (DLS) = 19.0 nm.

### Example 2. Adding an anchoring layer of insufficient thickness to precursor nanostructure - reference example outside the scope of the present disclosure

A 20 ml reaction vial equipped with a magnetic stir bar was charged with 10 ml of a solution of precursor nanostructures with an average diameter of 19.1 nm and a phosphorus concentration of 130 mM, formed by heating 5.74 g of 1,7-bis-(triethoxysilyl)-4,4-bis-(dimethylphosphonato)-heptane in 105 ml ethylene glycol and 26 ml water to 125 °C for 285 hours. The vial was heated in a preheated 100 °C oil bath for 10 minutes before 223 mg of 1,2-bis(triethoxysilyl)methane (0.65 mmol, 1 equivalent relative to the nominal amount of 1,7-bis-(triethoxysilyl)-4,4-bis-(dimethylphosphonato)-heptane in the solution) was added. The mixture was stirred vigorously for 5 minutes after which the solution was homogenous and the stirring speed lowered. After 4 hours of heating the vial was removed from the oil bath. Average diameter (DLS) = 20.2 nm. Thus, the thickness of the added anchoring layer was 0.55 nm.

### Example 3. Examples of unsuitable priming precursors - reference examples outside the scope of the present disclosure

### Example 3A. Adding a silica layer to precursor to form primed nanostructures having an anchoring layer formed from tetraethyl orthosilicate - reference example outside the scope of the current disclosure

A 20 ml reaction vial equipped with a magnetic stir bar was charged with 10 ml of a solution of precursor nanostructures with an average diameter of 19.1 nm and a phosphorus concentration of 130 mM, formed by heating 5.74 g of 1,7-bis-(triethoxysilyl)-4,4-bis-(dimethylphosphonato)-heptane in 105 ml ethylene glycol and 26 ml water to 125 °C for 285 hours. The vial was heated in a preheated 100 °C oil bath for 5 minutes before 673 mg of tetraethyl orthosilicate (3.23 mmol, 5 equivalents relative to the nominal amount of 1,7-bis-(triethoxysilyl)-4,4-bis-(dimethylphosphonato)-heptane) was added. The mixture was stirred vigorously for 10 minutes after which the solution was homogenous and the stirring speed lowered. After 4 hours of heating the vial was removed from the oil bath. Average diameter (DLS) = 21.5 nm. Thus, the thickness of the anchoring layer was 1.2 nm.

### Example 38. 1,6-bis(trimethoxysilyl)hexane does not form an anchoring laver-reference example outside the scope of the present disclosure

A 25 ml round bottom flask equipped with a magnetic stir bar was charged with 10 ml of a solution of precursor nanostructures with an average diameter of 16.2 nm and a phosphorus concentration of 71 mM, formed by heating 5.82 g of 1,7-bis-(triethoxysilyl)-4,4-bis-(dimethylphosphonato)-heptane in 250 ml 90.0 % (v/v) aqueous ethylene glycol reflux (140 °C) for 90 hours. The flask was heated in a preheated 100 °C oil bath for 15 minutes before 290 mg of 1,6-bis(trimethoxysilyl)hexane (0.89 mmol, 2.5 equivalents relative to the nominal amount of 1,7-bis-(triethoxysilyl)-4,4-bis-(dimethylphosphonato)-heptane) was added. The mixture was stirred vigorously for 10 minutes after which the solution was homogenous and the stirring speed lowered. Sampled for analysis after 30 minutes, 1 hour, 2 hours, and 3 hours. Aliquots of the samples diluted with water for DLS measurement could be filtered through 0.2 |jm syringe filters only with considerable force. DLS measurements of filtered samples showed no nanostructures in the range 5-150 nm, indicating essentially complete aggregation of the precursor nanostructures.

### Example 4. Adding a coating layer to primed nanostructures to form coated nanostructures

A 2-necked round bottom flask with the vertical neck equipped with a reflux condenser topped with a connection to a vacuum-nitrogen manifold and the side neck stoppered with a septum was charged with 5 ml of a solution of primed nanostructures prepared similarly to Example 1-b (average diameter (DLS)=22.2 nm; nominal [P]=130 mM; 0.65 mmol P; thickness of the anchoring layer = 1.5 nm). The nanostructure solution was degassed and lowered into a preheated 110 °C oil bath. After 10 minutes 400 µl of a 50 % (w/w) solution of 1,7-bis(triethoxysilyl)-4,4-bis(ω-methyl-(ethyleneoxy)₄₅-methyl)heptane in anhydrous dioxane heated to 40 °C was added. A further 2.7 ml of the warm 1,7-bis(triethoxysilyl)-4,4-bis(ω-methyl-(ethyleneoxy)₄₅-methyl)heptane solution was added by syringe pump at a speed of 142 pl/hour. After a total of 48 hours the solution was allowed to cool to ambient temperature. The solution was diluted to 25 ml with water and filtered through a 0.2 µm PES syringe filter. The resulting solution was diluted to 800 ml with water and concentrated to approximately 20 ml using a tangential flow filtration setup with a 300 kD nominal cut off. The dilution - concentration procedure was repeated for a total of three times before the solution was further concentrated to 7 ml on a spin filter with a 300 kD nominal cut off. Average diameter (DLS) = 29.6 nm. [P](ICP-OES) = 75 mM. [Si](ICP-OES) = 281 mM. Nanostructures prepared in this manner do not precipitate when exposed to a 20 mM CaCI2 solution, which is a factor that indicates compatibility with biological fluids.

### Example 5. Adding a coating layer to primed nanostructures with a priming layer of insufficient thickness - reference example outside the scope of the present disclosure

A 2-necked round bottom flask with the vertical neck equipped with a reflux condenser topped with a connection to a vacuum-nitrogen manifold and the side neck stoppered with a septum was charged with 4 ml of a solution of primed nanostructures prepared from Example 2 (average diameter (DLS) = 20.2 nm; nominal [P]=130 mM; 0.52 mmol P; thickness of the anchoring layer is 0.55 nm). The nanostructure solution was degassed and lowered into a preheated 110 °C oil bath. After 10 minutes 300 µl of a 50 % (w/w) solution of 1,7-bis(triethoxysilyl)-4,4-bis(ω-methyl-(ethyleneoxy)₄₅-methyl)heptane in anhydrous dioxane heated to 40 °C was added. A further 1.9 ml of the warm 1,7-bis(triethoxysilyl)-4,4-bis((ω-methyl-(ethyleneoxy)₄₅-methyl)heptane solution was added by syringe pump at a speed of 100 pl/hour. After a total of 48 hours the solution was allowed to cool to ambient temperature. The solution was diluted to 20 ml with water and filtered through a 0.2 µm PES syringe filter. The resulting solution was diluted to 800 ml with water and concentrated to approximately 20 ml using a tangential flow filtration setup with a 300 kD nominal cut off. The dilution - concentration procedure was repeated for a total of three times before the solution was further concentrated to 6.5 ml on a spin filter with a 300 kD nominal cut off. Average diameter (DLS) = 28.8 nm. [P](ICP-OES) = 76 mM. [Si](ICP-OES) = 175 mM. Nanostructures prepared in this manner do not precipitate when exposed to a 20 mM CaCI2 solution, but still show poor performance *in vivo* with a short plasma half-life as shown in Example 9.

### Example 6. Adding a coating layer to silica primed nanostructures having an anchoring layer formed from tetraethyl orthosilicate - reference example outside the scope of the present disclosure

A 2-necked round bottom flask with the vertical neck equipped with a reflux condenser topped with a connection to a vacuum-nitrogen manifold and the side neck stoppered with a septum was charged with 5 ml of a solution of primed nanostructures prepared according to Example 3A (average diameter (DLS)=21.5 nm, nominal [P]=130 mM; 0.65 mmol P). The nanostructure solution was degassed and lowered into a preheated 110 °C oil bath. After 5 minutes 400 µl of a 50 % (w/w) solution of 1,7-bis(triethoxysilyl)-4,4-bis(ω-methyl-(ethyleneoxy)45-methyl)heptane in anhydrous dioxane heated to 40 °C was added. A further 2.7 ml of the warm 1,7-bis(triethoxysilyl)-4,4-bis(ω-methyl-(ethyleneoxy)45-methyl)heptane solution was added by syringe pump at a speed of 142 pl/hour. After a total of 48 hours the solution was allowed to cool to ambient temperature. The solution was diluted to 25 ml with water and filtered through a 0.2 µm PES syringe filter. The resulting solution was diluted to 800 ml with water and concentrated to approximately 20 ml using a tangential flow filtration setup with a 300 kD nominal cut off. The dilution - concentration procedure was repeated for a total of three times before the solution was further concentrated to 6 ml on a spin filter with a 300 kD nominal cut off. Average diameter (DLS) = 29.4 nm. [P](ICP-OES) = 83 mM. [Si](ICP-OES) = 325 mM. Nanostructures prepared in this manner do not precipitate when exposed to a 20 mM CaCI2 solution but show poor hydrolytic stability as shown in Example 8.

### Example 7. Adding a coating layer to non-primed nanostructures - reference example outside the scope of the present disclosure

A 2-necked round bottom flask with the vertical neck equipped with a reflux condenser topped with a connection to a vacuum-nitrogen manifold and the side neck stoppered with a septum was charged with 5 ml of a solution of non-primed precursor nanostructures with an average diameter of 19.1 nm and a phosphorus concentration of 130 mM, formed by heating 5.74 g of 1,7-bis-(triethoxysilyl)-4,4-bis-(dimethylphosphonato)-heptane in 105 ml ethylene glycol and 26 ml water to 125 °C for 285 hours. The nanostructure solution was degassed and lowered into a preheated 110 °C oil bath. After 5 minutes 400 µl of a 50 % (w/w) solution of 1,7-bis(triethoxysilyl)-4,4-bis(ω-methyl-(ethylene0xy)as-methyl)heptane in anhydrous dioxane heated to 40 °C was added. A further 2.7 ml of the warm 1,7-bis(triethoxysilyl)-4,4-bis(ω-methyl-(ethylene0xy)as-methyl)heptane solution was added by syringe pump at a speed of 142 pl/hour. After a total of 48 hours the solution was allowed to cool to ambient temperature. The solution was diluted to 25 ml with water and filtered through a 0.2 µm PES syringe filter. The resulting solution was diluted to 800 ml with water and concentrated to approximately 20 ml using a tangential flow filtration setup with a 300 kD nominal cut off. The dilution - concentration procedure was repeated for a total of three times before the solution was further concentrated to 6 ml on a spin filter with a 300 kD nominal cut off. Average diameter (DLS) = 29.0 nm. [P](ICP-OES) = 80 mM. [Si](ICP-OES) = 107 mM. Nanostructures prepared in this manner precipitate when exposed to a physiologically relevant 2 mM CaCI2 solution, demonstrating less suspension stability of coated nanostructures lacking an anchoring layer as compared to coated nanostructures comprising an anchoring layer according to the present disclosure (Example 4).

### Example 8. Comparison of hydrolytic stability

A 1.25 ml sample of the nanostructure solution from Example 4 was diluted to 4 ml with water. To this solution was added 204 µl of a 19.7 mM solution of LuCl₃ in water and the solution was heated to 60 °C before addition of 1.26 ml of a 1 M Tris buffer with a pH of 7.7. Heating was continued for 2 hours and the resulting solution was filtered through a 0.2 µm syringe filter.

A sample of the nanostructure solution from Example 6 was treated with LuCI3 in the same manner.

Samples of these solutions were then subjected to constant volume tangential flow filtration in 75 mmol Tris buffer with a pH of 7.4 with a filter with a 50 kD nominal cutoff for 20 hours and the retentate was analyzed for [Si] and[P] by ICP-OES. The ratio of [Si]/[P] decreased from 3.71 to 3.66 (-1 %) for the nanostructure solution from Example 4 and from 3.74 to 3.23 (-14 %) for the nanostructures from Example 6, demonstrating the higher hydrolytic stability of nanostructures with an anchoring layer derived from BTESM (Example 4).

### Example 9. Comparison of in-vivo stability

Solutions of nanostructures from Examples 4, 5, and 6 loaded with lutetium (LuCI3) as in Example 8 were further formulated for injection by addition of CaCI2, HCI, and mannitol to result in injection solutions with pH=7.4, [P]=9-11 mM, and [Lu]=0.40 mM and an osmolality suitable for injection.

*In vivo* behavior was investigated by injecting female BALB/c mice with the test items at a dose of 2 µmol Lu/kg. Blood was sampled at 1, 6, and 24 hours after injection and plasma was analyzed for [Lu] and [Si] by ICP-OES.

The plasma t_{½} (plasma half-life) for the nanostructures, as calculated from the concentration of Lu, was a beneficially long 12 hours for the nanostructures from Example 4 (having a thickness of the anchoring layer = 1.5 nm), 5 hours for the nanostructures from Example 5 (having a thickness of the anchoring layer = 0.55 nm), and 4 hours for the nanostructures from Example 6 (having an anchoring layer formed from tetraethyl orthosilicate) demonstrating the increased plasma half-life *in vivo* of nanostructures with an anchoring layer according to the present disclosure, here demonstrated for an anchoring layer derived from BTESM, if said anchoring layer is thick enough.

If the t_{½} was calculated from the concentration of Si, the result is still 12 hours for the nanostructures from Example 4 (having an anchoring layer according to the present disclosure), but only 5 h for the nanostructures of Example 5, and 3 hours for the nanostructures from Example 6, demonstrating the higher hydrolytic stability of nanostructures with an anchoring layer according to the present disclosure, here demonstrated for an anchoring layer derived from BTESM *in vivo.*

### Example 10. NMR of primed nanostructures

Samples of precursor nanostructures similar to the ones used in Example 1B and of primed nanostructures similar to the product from Example 1B were transferred to D₂O by first exchanging the solvent to water using repeated dilution-concentration cycles on a 100 kD nominal cut-off spin filter and then exchanging the water for D₂O in the same manner.

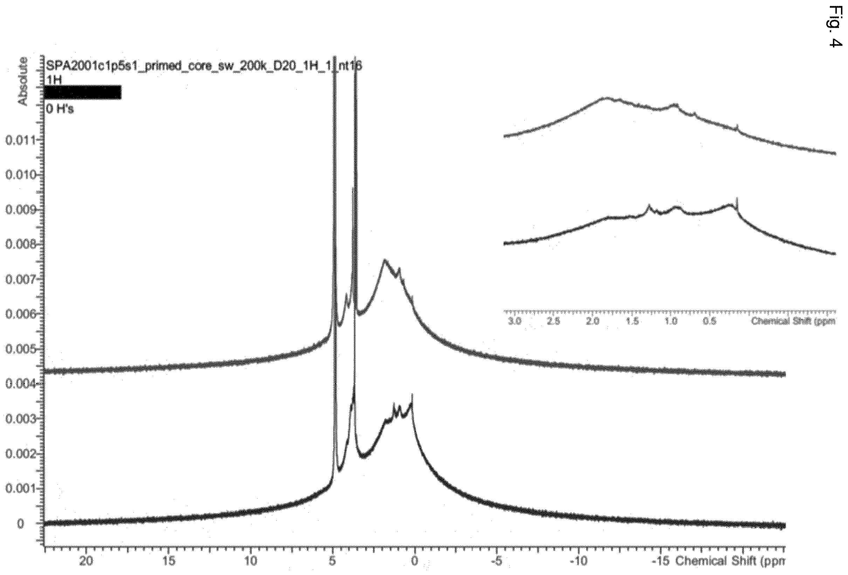

1H-NMR spectra were recorded and are shown in Figure 4. The upper spectrum is the spectrum for the precursor nanostructures offset from the spectrum for the primed nanostructures for clarity. The inset displays a zoom-in of the same spectra, showing salient features in the region around 0 ppm.

The main peak for the precursor nanostructures is extremely broadened, with a peak with of ca 25 ppm (12500 Hz), in accordance with a high degree of cross-linking in the precursor nanostructures. The spectrum for the primed nanostructures displays essentially the same features, except for a smaller, but similarly broadened, peak centered at 0.25 ppm corresponding to the methylene moiety in the anchoring layer. This demonstrates a high degree of cross-linking in the anchoring layer.

### Example 11. Synthesis of nanostructures with different sizes and thickness of the anchoring layer

Precursor nanostructures of various sizes, synthesized in similar fashion as the precursor nanostructures in examples 1A and 1B, were treated with BTESM similarly to Example 1b, but with varying amounts of BTESM according to Table 1. The diameters of the primed nanostructures were evaluated by DLS. As can be seen from Table 1, many combinations of core (precursor nanostructure) and anchoring layer can be achieved in a predictable way.

**Table 1. Precursor nanostructure diameter and primed nanostructure diameter for some precursor nanostructures between 12.5 and 23 nm, treated with between 0.5 and 7 equivalents of BTESM**

| **Example** | **Precursor nanostructure diameter (nm)** | **Number of equivalents of anchoring layer precursor** | **Primed nanostructure diameter (nm)** |
|---|---|---|---|
| **11a** | 15.2 | 0.5 | 17.5 |
| **11b** | 12.5 | 2.5 | 13.8 |
| **11c** | 14.3 | 2.5 | 16.4 |
| **11d** | 15.2 | 2.5 | 19.6 |
| **11e** | 17.2 | 2.5 | 19.9 |
| **11f** | 18.3 | 2.5 | 21.1 |
| **11g** | 19.1 | 2.5 | 22.5 |
| **11h** | 23.0 | 2.5 | 27.3 |
| **11i** | 12.5 | 3 | 13.7 |
| **11j** | 12.5 | 5 | 14.0 |
| **11k** | 12.5 | 7 | 14.4 |

### Example 12. Calculation of density and degree of crosslinking of the anchoring layer.

Samples of primed nanostructures prepared similarly to Example 1a and of precursor nanostructures similar to the ones used in Example 1a were dried to a powder by first exchanging the solvent to water by a procedure similar to the one used in example 10, and then freeze-drying the resulting solution.

The density of the resulting powders was determined by constricting it between the density of a mixture of heptane and dibromomethane such that the powder barely sediments on centrifugation and the density of a mixture of heptane and dibromomethane such that the powder barely floats on centrifugation. The densities were found to be 1.739 g/ml and 1.649 g/ml for the primed nanostructures and the precursor nanostructures, respectively. The density of the anchoring layer was calculated to 1.9 g/ml (1.82-2.35 g/ml) by assuming a core-shell model (se Fig. 2B) and spherical nanostructures.

This indicates an average degree of cross-linking of about 4 bonds to the polymeric network per monomer residue.

## Claims

1. A plurality of globular nanostructures, wherein the plurality of globular nanostructures has a dispersity between 1 and 1.8; and wherein each nanostructure comprises:
a central part comprising a polymer of monomer residues, wherein the central parts of the nanostructures have a volume average hydrodynamic diameter of 10 nm to 90 nm; and
an anchoring layer surrounding the central part, wherein the anchoring layer comprises a polymer of monomer residues according to Formula (I)
(RO)₃Si-X-Si(OR)₃ (I)
wherein
each R is independently selected from the group consisting of a negative charge, H and a covalent bond, wherein at least two R are independently selected from the group consisting of a covalent bond to a monomer residue of the central part and a covalent bond to a monomer residue of the anchoring layer; and
X is -(CH₂)ₙ-, wherein - represents a covalent bond and n is an integer between 1 and 5; and
wherein the anchoring layer has a thickness of 1-5 nm.

2. A plurality of nanostructures according to claim 1, wherein the anchoring layer has a thickness of 1.1-2.5 nm, or 1.3-1.6 nm.

3. A plurality of nanostructures according to any one of the preceding claims, wherein 2 to 5 of the R-groups are selected from the group comprising a covalent bond to a monomer residue of the central part and a covalent bond to a monomer residue of the anchoring layer.

4. A plurality of globular nanostructures according to any one of the preceding claims, wherein n is 1 or 2.

5. A plurality of globular nanostructures according to any one of the preceding claims, wherein the central part comprises a polymer framework of monomer residues according to Formula (II)
{(OR¹)(OR²)PO}₂-(C){(CH₂)ₘSi(OR³)₃}{(CH₂)ₘSi(OR³)₃} (II)
wherein
each R¹ and R² is independently selected from the group consisting of a negative charge and H;
each R³ is independently selected from the group consisting of a negative charge, H and a covalent bond to the polymeric framework; wherein at least 3 R³ are bonds to the polymeric framework; and
m is an integer between 1 and 5.

6. A plurality of globular nanostructures according to any one of the preceding claims, wherein the nanostructures further comprise a coating, preferably wherein the coating comprises hydrophilic groups.

7. A plurality of globular nanostructures according to claim 6, wherein the coating comprises polyethylene glycol.

8. A method for producing a plurality of globular nanostructures according to anyone of claims 1 to 7, comprising the steps of:
(1) providing a mixture of precursor nanostructures having a volume average hydrodynamic diameter of 10 nm to 90 nm, each precursor nanostructure comprising a polymer of monomer residues, and monomers according to Formula (III)
Si{(OR¹)(OR²)(OR³))-(CH₂)n-Si{(OR⁴)(OR^{S})(OR⁶)} (III)
wherein
R¹, R², R³, R⁴, R⁵, and R⁶ are independently selected from the group consisting of lower alkyls and aryl; and
n is an integer between 1 and 5;
in a mixture of water and a water miscible organic solvent; and
wherein the ratio of monomer residues of the precursor nanostructures to monomers according to Formula (III) is 1:0.5 to 1:20; and
(2) heating the mixture of step (1) to a temperature between 20 °C and 150 °C for a time period of 1 to 24 h.

9. The method according to claim 8, wherein R¹, R², R³, R⁴, R⁵, and R⁶ are independently selected from the group consisting of methyl and ethyl; and wherein n is 1 or 2.

10. The method according to claim 8 or claim 9, wherein the ratio of monomer residues of the precursor nanostructures to monomers according to Formula (III) is 1:0.5 to 1:7; and/or wherein, in step (2), the mixture of step (1) is heated to a temperature between 80 °C and 150 °C for a time period of 2 to 6 h.

11. A pharmaceutical composition comprising a plurality of globular nanostructures according to claim 6 or claim 7.

12. A pharmaceutical composition for use in the treatment of cancer and/or in imaging, wherein the pharmaceutical composition comprises a plurality of globular nanostructures according to claim 6 or claim 7, wherein the globular nanostructures further comprise a radioactive isotope.

13. Use of a plurality of globular nanostructures according to any of the claims 1 to 7 and/or the product of the method according to any one of claims 8 to 10, as an intermediate in the production of a plurality of globular coated nanostructures.

14. Use of the plurality of globular nanostructures according to any one of claims 1 to 7 and/or the product of the method according to any one of claims 8 to 10, as an intermediate in the production of a pharmaceutical product.

15. Use of a pharmaceutical composition according to claim 11 as a carrier of a radioactive isotope.
